Europäisches Patentamt

(19) European Patent Office

Office européen des brevets

(11) EP 0 673 239 B1

(12) EUROPEAN PATENT SPECIFICATION

(45) Date of publication and mention
of the grant of the patent:
**10.06.1998 Bulletin 1998/24**

(21) Application number: **94900982.3**

(22) Date of filing: **08.12.1993**

(51) Int. Cl.⁶: **A61K 9/00**

(86) International application number:
**PCT/IE93/00058**

(87) International publication number:
**WO 94/13261 (23.06.1994 Gazette 1994/14)**

(54) **AQUEOUS ANTIBIOTIC COMPOSITION FOR VETERINARY USE**

WÄSSERIGE ANTIBIOTISCHE ZUSAMMENSETZUNG ZU VETERINÄREM GEBRAUCH

COMPOSITION ANTIBIOTIQUE AQUEUSE POUR APPLICATION VETERINAIRE

(84) Designated Contracting States:
**DE DK FR NL**

(30) Priority: **08.12.1992 IE 922872**

(43) Date of publication of application:
**27.09.1995 Bulletin 1995/39**

(73) Proprietor:
**BIMEDA RESEARCH AND DEVELOPMENT
LIMITED
DUBLIN 24 (IE)**

(72) Inventors:
• **CORRIGAN, Owen
County Dublin (IE)**
• **MORGAN, James Patrick
County Meath (IE)**

• **LOGUE, Paul
Dublin 6 (IE)**
• **O'LEARY, John
Booterstown, County Dublin (IE)**

(74) Representative:
**O'Brien, John Augustine et al
John A. O'Brien & Associates,
Third Floor,
Duncairn House,
14 Carysfort Avenue
Blackrock, Co Dublin (IE)**

(56) References cited:
**EP-A- 0 271 306          FR-A- 2 230 339
GB-A- 1 441 747**

## Description

The invention relates to a veterinary composition, particularly for the prophylaxis and treatment of mastitis in cows.

More particularly, the invention relates to a veterinary composition for intramammary use in non-human animals comprising cloxacillin benzathine as an antibacterial agent.

Bacterial infection via the teats of a cow is the most common cause of mastitis.

In one aspect the invention relates to a veterinary composition for treating mastitis in dry cows.

It is known to treat teats of a cow with a long acting antibiotic with effective cover only being provided whilst minimum inhibitory concentration (MIC) levels of the antibiotic are maintained. This period of cover can vary from 4 to 10 weeks.

FR-A-2230339 describes a method for prophylaxis or treatment of mammary disorders during the animal's dry period by administering an antibacterial agent in admixture with an oil insoluble and water insoluble binding agent.

EP-A-0271306 describes a method for the treatment of mammary disorders in non-human animals comprising administering an antibacterial agent to the animal by the intramammary route. The antibacterial agent is administered in the form of particles suspended in a hydrophobic oily vehicle comprising oil and gelling agent.

It is also known to provide a physical barrier in the teat canal to try to prevent the ingress of pathogens. One such system is described in UK 1,441,747 (Lazonby).

One commercially available barrier system comprises a twin injector pack, one injector containing an antibiotic formulation and a second injector containing a barrier or seal formulation. The antibiotic formulation comprises penicillin salts and dihydrostreptomycin which is infused into the udder following the last lactation and before the cow has dried off. The seal formulation comprises a gel of aluminium stearate and liquid paraffin containing approximately 35% by weight of bismuth subnitrate. This is infused into the udder after the antibiotic formulation to seal the teat canal.

In another aspect the invention relates to a veterinary composition for treating mastitis in lactating cows.

It is known to treat mastitis in lactating cows with antibiotics suspended in non-aqueous bases. The antibiotics are generally administered at either 12 or 24 hour intervals over a period of days. This is not only inconvenient for the farmer or veterinary surgeon but is also stressful to the animal and increases the risk of further infection. There is a further problem in that any milk delivered from an affected cow must be withheld from human consumption for a period of up to 144 hours from the commencement of treatment.

This invention is therefore directed towards providing an improved veterinary composition for the prophylaxis and treatment of mastitis in dry cows and lactating cows.

The invention is characterised in that the composition comprises 600 mg of cloxacillin (as cloxacillin benzathine) per unit dose having an average dimension of less than 25 microns in an aqueous suspension including a suspension aid, a buffer, and a surfactant.

Preferably a substantial proportion of the antibiotic has an average dimension of less than 10 $\mu$.

In one embodiment of the invention the composition includes a suspension aid polyvinylprollidone. In another embodiment of the invention the composition includes a buffer sodium citrate.

In a preferred arrangement the composition includes a surfactant.

Most preferably the composition is in the form of an injector for intramammary administration.

The composition is especially for use in the prophylaxis or treatment of mammary disorders in lactating animals.

The invention also provides a veterinary product for intramammary use in non-human animals during an animals' dry period, the product comprising a veterinary composition according to the invention and a gel base seal formulation.

Preferably, a heavy metal salt is present in the gel base. Typically, the heavy metal salt is present in an amount of at least 40% by weight of the base, preferably between 50% and 75% by weight, most preferably approximately 65% by weight.

In one embodiment of the invention the heavy metal salt is bismuth sub-nitrate.

In one embodiment of the invention the base is a gel based on aluminium stearate. In this case preferably the gel includes a vehicle such as liquid paraffin.

In another embodiment of the invention the gel comprises a polyethylene gel. The gel may be based on low density polyethylene or on high density polyethylene.

## Detailed Description of the Invention

The invention will be more clearly understood from the following description thereof given by way of example only.

## EXAMPLE A - LC

A single dose veterinary composition comprising an antibiotic-containing injector for treating mastitis in lactating cows was prepared as follows :

**INJECTOR 1A**

| Component | g/Kg | Function |
|---|---|---|
| *Cloxacillin Benzathine | 212.6 | Antibiotic |
| PVP | 0.59 | Suspension Aid |
| Sodium Citrate | 7.87 | Buffer |
| Tween 80 | 0.983 | Surfactant |
| EDTA (disodium) | 0.0787 | Cation Scavenger |
| Antifoam M30 | 0.0157 | Production Aid |
| Water for Injection | QS | Aqueous Vehicle |

\*
(i) will be adjusted depending on potency.
(ii) the cloxacillin benzathine was in a micronised form having an average dimension less than $25\mu$ with approximately 75% less than $15\mu$ and 50% less than $10\mu$ and 85% was greater than $2\mu$.

(1) Place most of the water for injection in a production vessel.
(2) Add and dissolve separately, sodium citrate, E.D.T.A., P.V.P. and Tween 80. Mix well.
(3) Add antifoam and mix well, the solution will have a slight haze.
(4) Add and suspend Cloxacillin Benzathine and homogenise for 15 minutes.
(5) Bring to final weight with addition of further water for injection.
(6) Fill 3.6g into intramammary injectors.

All of the above steps are carried out under normal aseptic conditions.

This formulation is stable when subjected to extended storage periods in its proposed marketing container.

We have surprisingly found that once infused by the intramammary route cloxacillin benzathine in an aqueous base gives rapid absorption in a very short time period.

A number of studies have been conducted in cows to establish the following : -

**Study 1:** Bioavailability of cloxacillin in bovine colostrum following intramammary infusion of Injector 1A.
**Study 2:** Irritancy of aqueous cloxacillin in the bovine mammary gland.

The results can be summarised as follows.

**INJECTOR 1A - Study 1**

The primary objective of this study was to demonstrate the bioavailability of cloxacillin in the bovine udder after the infusion of an aqueous formulation of Cloxacillin Benzathine. As a secondary objective plasma levels of Cloxacillin were also measured, in order to assess the extent of systemic absorption.

Eight animals were infused with Injector 1A in all four quarters, and samples of milk were taken at the following intervals post infusion : 0.5, 1, 2, 3, 4, 6, 8, 12, 24, 32, 48, 72, 96, 120 and 144 hours.

Quarter results were recorded for all animals (it was not possible to get samples from two of the quarters due to problems associated with hand milking of animals normally used to being machine milked). Not surprisingly there was a large variation in the amount of drug found, even between quarters of the same animal. A peak mean value of 5233.4 mg/L was found at the first sampling interval (0.5 hr) with an approximately exponential decline being noted over the next 72 hours. The results indicate that the antibiotic was extensively distributed throughout the mammary gland, as significant amounts of drug were still found at 24, 32 and 48 hours post the initial infusion, despite the removal of drug at each milking.

In addition, the level of a systemic absorption of Cloxacillin was assessed by monitoring levels of drug in the plasma. Sampling intervals were identical to those for the milk study except that a further four samples were taken at 168, 192, 216 and 240 hours post infusion.

The amount of drug detected in the plasma of treated animals peaked at between 6-8 hours at a mean of 0.15 mg/L. The highest level seen in any animal was 0.22 mg/L. By 96 hours post infusion drug was detectable in one animal only, and it continued to be detectable in that animal for a further 144 hours. This animal also had drug present in its pre-dose plasma sample and would seem to be somewhat of an outlier. However, as no drug was detected in its pre-dose milk sample nor was it any different in its elimination of drug from the milk, it was considered valid to include it in the set of results.

This study indicates that a Cloxacillin Benzathine aqueous formulation has the desired characteristics of producing rapid absorption and distribution within the udder, producing large peak concentrations of drug followed by a swift removal of the antibiotic from the bovine mammary gland, as evidenced by the fact that no drug was detectable in the quarter of any animal 144 hours post infusion.

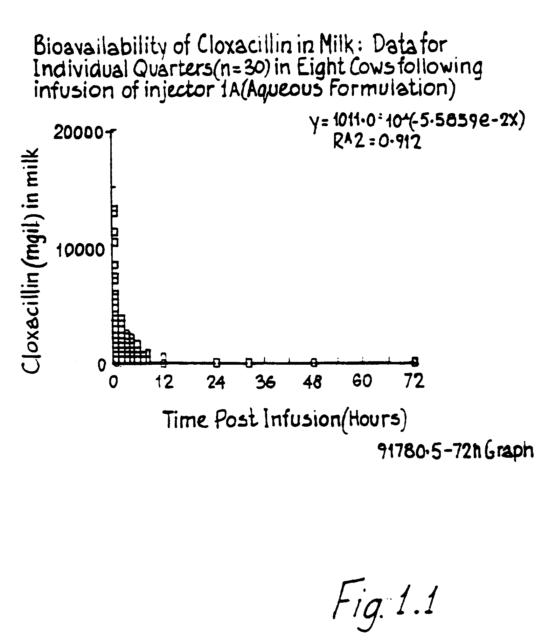

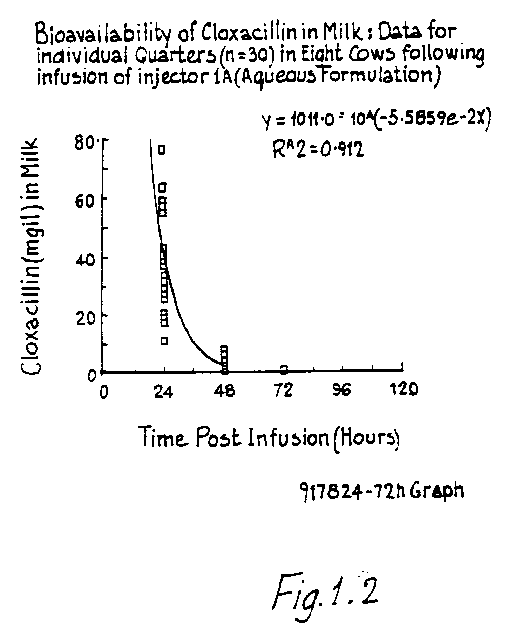

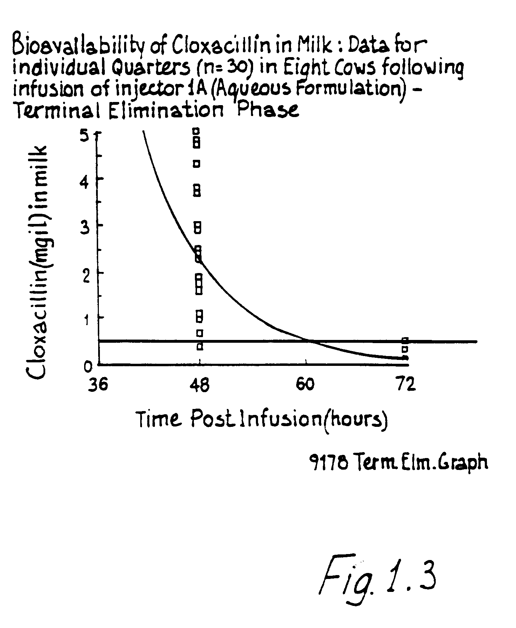

Figure 1.1, 1.2 and 1.3 are graphic representations of the results of this study.

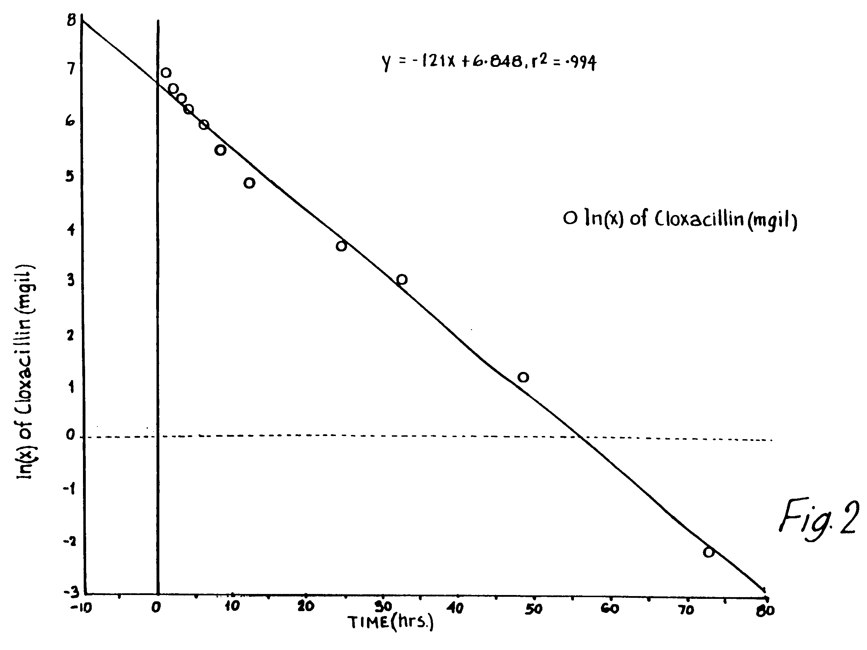

Fig 2: is a log transformation of the results of Figure 1.1.

## Study 2.

Injector 1A was mildly irritant to the bovine udder when used as an intramammary infusion. However, any rise in somatic cell counts were transitory in nature and all animals had returned to normal within 72 hours of the infusion of the product. Hence it can concluded that this formulation is suitable for use as a lactating intramammary.

By providing an insoluble salt of a synthetic penicillin such as cloxacillin benzathine in a micronised form in an aqueous base we have achieved in a single dose a high initial peak of antibiotic within 30 minutes of administration which is maintained at therapeutic levels for up to 72 hours. Within 30 minutes the antibiotic administered also crosses the blood-milk barrier establishing a drug reservoir which aids the maintenance of therapeutic levels of cloxacillin for the 72 hour treatment period. This is particularly important where there is a systemic involvement in the mastitic condition. All the above is achieved with a single dose.

## EXAMPLE A -DC

A veterinary composition for treating mastitis in dry cows was prepared. The twin-pack composition comprised an antibiotic-containing injector and a seal-containing injector. The antibiotic-containing injector was the same as the injector 1A described above. The injector 1A is suitable for dry cow therapy in association with a teat seal, providing as it does high initial peaks of cloxacillin followed by a rapid elimination phase of the drug. Studies 1 and 2 above confirm this. The following additional study was also carried out specifically for dry cow applications to assess the residues of cloxacillin in bovine colostrum following intramammary infusion of the antibiotic injector. The second seal-containing injector (2A) is described below.

## INJECTOR 1A - Study 3

Study 3 was conducted to specifically determine the end point for milk withholding. Animals were infused in each of four quarters with the Injector 1A. Samples were taken every 24 hours and analysed for cloxacillin levels. Eight days after administration of Injector 1A the levels of drug were below the acceptable maximum residue level for cloxacillin.

## INJECTOR TYPE 2A

Various gels based on liquid paraffin with aluminium stearate were prepared.

| Formulation | Mass | Constituents | YV/(Nm$^{-2}$) |
|---|---|---|---|
| 2A1 | 3.5g | 14% AS-LP gel - 37%BSN + 0.1%Ac | 110.3 |
| 2A2 | 7.0g | 14% AS-LP gel - 37%BSN + 0.1%Ac | 110.3 |

(continued)

| Formulation | Mass | Constituents | YV/(Nm$^{-2}$) |
|---|---|---|---|
| 2A3 | 3.5g | 14% AS-LP gel - 37%BSN + 0.1%Ac | 215.5 |

| LP = Liquid Paraffin |
| BSN = Bismuth Subnitrate |
| Ac = Acriflavin |
| AS = Aluminium Monostearate |
| YV = Yield value - (A measure of the relative fluidity of the gel. Low yield values indicate a more liquid gel). |
| Products 2A1 to 2A3 were considered appropriate candidates for use as test seals. |

An ideal teat seal should have the following characteristics :

1. It should be non-irritant to the bovine udder;

2. Persistence - the seal should remain in situ for the duration of the dry cow period;

3. Consistency - the seal should not break up within the udder;

4. Compatibility - the seal should be compatible with the antibiotic formulation used in association with it, either aqueous or oily;

5. Ease of Removal - at the end of the dry period, the seal should be readily removable for the udder and not give rise to persistent residues of either the seal or antibiotic.

Irritancy of the seals was the first characteristic to be assessed as any product which was irritant would have to be rejected irrespective of its performance against the other criteria. Irritancy was measured by conducting somatic cell counts in treated and untreated quarters of lactating cows and comparing these results by measuring area under the curve ratios using the following formula:

$$\text{AUC Ratio} = \frac{\text{AUC of treated quarter}}{\text{AUC of untreated quarter}}$$

This allows for a relative assessment of the various seal formulae.

| Formulation | AUC Ratio | Peak (cells/ml) | Condition of milk | Duration before return to pre-dose level (hours) |
|---|---|---|---|---|
| 2A1 | 1.25 | $9.0 \times 10^5$ | Normal | 160 |
| 2A2 | 1.33 | $1.0 \times 10^6$ | Normal | 144 |
| 2A3 | 1.28 | $8.5 \times 10^5$ | Normal | 160 |

Formulations 2A1 and 2A3 were considered appropriate for further investigation. It was concluded that AS-LP gels are viable candidates for sealing teats.

In vitro studies have shown that whilst AS-LP gels have relatively high yield values, their tensile strength is not as great as other possible seal formulations. The relative merits of these two properties were studied by X-ray analysis of various formulations in dry cows.

A series of studies were undertaken to optimise these parameters :

**INJECTOR TYPE 2A - Study 1**

| Formulation | Gel former % | LP % | AC % | BSN % | Mass(g) | P g cm$^3$ | YV Nm$^{-2}$ |
|---|---|---|---|---|---|---|---|
| 2A1 | 8.8 AS | 54.1 | 0.1 | 37 | 7.0 | 1.32 | 110.0 |

| AS = Aluminium Stearate |
|---|
| LP = Liquid Paraffin |
| AC = Acriflavin |
| BSN = Bismuth Subnitrate |
| p = Density |
| YV = Yield Value |

The test formulation was infused into quarters of dry cows. The effectiveness of sealing was measured by X-ray analysis. In addition the mass of seal recovered, % BSN recovered and the effective seal duration [ESD] were estimated.

| Formulation | ESD (days) | Mass of seal recovered (%) | BSN recovered |
|---|---|---|---|
| 2A | 28.5 ± 13.1 | 8.19 (117.1) | 64.1 |

The mass of seal recovered was in excess of that applied probably due to the presence of aqueous material.

**INJECTOR TYPE 2A - Study 2**

The effect of the product volume and density was examined in another series of X-ray studies.

| Formulation | Gel former % | LP % | AC % | BSN % | Mass g | P g cm$^3$ | YV Nm$^{-2}$ |
|---|---|---|---|---|---|---|---|
| 2A4 | 3.1 AS | 31.8 | 0.1 | 65 | 5.0 | 1.70 | 161.7 |
| 2A5 | 3.1 AS | 31.8 | 0.1 | 65 | 10.0 | 1.70 | 161.7 |

The results of these studies are shown in the table below:

| Formulation | Mass(g)* recovered (%) | % BSN ** recovered |
|---|---|---|
| 2A4 | 2.47 (49.4) | 89.8 |
| 2A5 | 4.52 (45.2) | 100.2 |

* Adjusted for water content
** % BSN were higher than in the previous study indicating a greater integrity of seal. As this trial was of a shorter duration than study 1, this may have been a contributing factor. However, increasing the density and reducing the volume had a clearly beneficial effect on the product performance.

**INJECTOR TYPE 2A - Study 3**

As it is intended to use the teat seal in conjunction with an antibiotic preparation this study looked at the effect of an aqueous and oily based antibiotic suspension on seal integrity over time. Additionally, the mass of seal was reduced to 3.5g to see if this could further enhance the seals effectiveness.

Products used are given in the table below :

| Formulation | Gel former % | LP % | Ac. % | BSN % | Mass g | P g/cm$^3$ | YV NM$^{-2}$ | Antibiotic |
|---|---|---|---|---|---|---|---|---|
| 2A6 | 3.1% AS | 31.8 | 0.1 | 65 | 3.5 | 1.70 | 161.7 | Oily |
| 2A6$^1$ | 3.1% AS | 31.8 | 0.1 | 65 | 3.5 | 1.70 | 161.7 | Aq. |

The products were then examined for their effective seal duration [ESD].

| Formulation | ESD (days) | Recovery of material (g) | BSN Recovery % | BSN recovery in fluid portion ppm |
|---|---|---|---|---|
| 2A6 | 47.8 ± 13.5 | 0 | 0 | 2191 |
| 2A6$^1$ | 63.3 ± 5.4 | 2.06 | 75.2 | 117 |

A combination of a seal and an aqueous based antibiotic offers a superior combination to a seal and an oily based antibiotic. This can be deduced from the fact that the ESD's for the seal/aqueous combinations were significantly better than those for oily combination. This is further evidenced by the high BSN recoveries (>75%) for the aqueous products, indicating retention of seal in situ. In contrast, the amount of bismuth found in the fluid portion at parturition was far lower for the aqueous than the oily combination. This shows that there was a larger degree of dispersal of the seal into the udder with the oily combinations.

The amount of BSN lost in absolute terms can be calculated as follows :

$$A \times B \times M = \text{Loss of BSN (g)}$$

A = % BSN lost.
B = % of BSN in product
M = Mass of Product

Using the formula, product 2A1 lost 1.30 g. 2A6$^1$ lost 0.57g. Considering that product 2A6$^1$ was in situ for 70 days as compared to 49 days for 2A1, this demonstrates at least a 2-3 fold reduction in BSN loss and a major improvement in product design.

**PREFERRED METHOD OF MANUFACTURE FOR INJECTOR TYPE 2A**

| | Gm/Kg |
|---|---|
| Aluminium Stearate (Alugel 30 D.F.) | 48.9 |
| Heavy Liquid Paraffin | 300.4 |
| Bismuth Subnitrate | 650.00 |
| Acriflavin (Pigment) | 0.994 |

Each injector contains 3.5g.

The aluminium stearate used is a distearate compound having a melting point in the region of 150°C to 160°C. Bismuth subnitrate ($6Bi_2O_3.5N_2O_5.9H_2O$) is a white, slightly hydroscopic powder.

(1) Place heavy liquid paraffin in reactor vessel. heat to 160°C for one hour. Cool to 40°C.

(2) Start emulsifiers and mixers and add the aluminium stearate. Heat to 145°C ± 5°C and maintain for one hour. Cool to 40°C.

(3) Add and blend the Bismuth Subnitrate and Acriflavin.

(4) Fill 3.5 g into intramammary injector.

EXAMPLE B

A veterinary composition was prepared comprising a first antibiotic-containing injector having the same formulation as Injector 1A described above and a second seal injector 2B as described below.

**INJECTOR TYPE 2B**

Various gel based on liquid paraffin with polyethylene were prepared. Two grades of polyethylene were used in manufacturing the gels: low density (LDPE) and high density (HDPE). They differed in the degree of side chain branching but produced similar gels.

| Formulation | Mass | Constituents | YV ($NM^{-2}$) |
|---|---|---|---|
| 2B1 | 3.5g | 3% HDPE - LP gel + 37% BSN + 0.1%Ac | 40.9 |
| 2B2 | 7.0g | 3% HDPE - LP gel + 35% BSN + 0.1%Ac | 40.9 |
| 2B3 | 7.0g | 5% HDPE - LP gel + 37% BSN + 0.1%Ac | 110.0 |
| 2B4 | 7.0g | 5% HDPE - LP gel + 37% BSN + 0.1%Ac | 220.3 |
| 2B5 | 3.5g | 3% HDPE - LP gel + 37% BSN + 0.1%Ac | 65.8 |
| 2B6 | 7.0g | 3% HDPE - LP gel + 37% BSN* + 0.1%Ac | 36.6 |
| 2B7 | 7.0g | 3% LDPE - LP gel + 37% BSN* + 0.1%Ac | 54.1 |

LP = Liquid Paraffin
BSN= Bismuth Subnitrate
Ac = Acriflavin
YV = Yield value - (A measure of the relative fluidity of the gel. Low yield values indicate a more liquid gel).

\* = BSN was in micronised form.

Products 2B1 and 2B7 were considered appropriate candidates for use as test seals. An ideal teat seal should have the following characteristics:

1. It should be non-irritant to the bovine udder;

2. Persistence - the seal should remain in situ for the duration of the dry cow period;

3. Consistency - the seal should not break up within the udder;

4. Compatibility - the seal should be compatible with the antibiotic formulation used in association with it, either aqueous or oily;

5. Ease of Removal - at the end of the dry period, the seal should be easily removable for the udder and not give rise to persistent residues of either the seal or antibiotic.

Irritancy of the seals was the first characteristic to be assessed as any product which was irritant would have to be rejected irrespective of its performance against the other criteria. Irritancy was measured by conducting somatic cell counts in treated and untreated quarters of lactating cows and comparing these results by measuring area under the curve ratios using the following formula:

$$\text{AUC Ratio} = \frac{\text{AUC of treated quarter}}{\text{AUC of untreated quarter}}$$

This allows for a relative assessment of the various seal formulae.

| Formulation | AUC RATIO | PEAK (CELLS/mL) | CONDITION OF MILK | DURATION BEFORE RETURN TO PRE-DOSE LEVEL (HOURS) |
|---|---|---|---|---|
| 2B1 | 17.0 | $5.3 \times 10^6$ | N | 160 |
| 2B2 | 4.8 | $2.4 \times 10^6$ | N | 160 |
| 2B3 | 16.4 | $>3 \times 10^6$ | N | 184 |
| 2B4 | 3.7 | $1.7 \times 10^6$ | N | 136 |
| 2B5 | 10.8 | $1.0 \times 10^6$ | N | 112 |
| 2B6 | 13.0 | $7.0 \times 10^6$ | N | 120 |
| 2B7 | 51.8 | $>1.0 \times 10^7$ | C | 112 |
| N = Normal C = Clotted | | | | |

Formulations 2B1 and 2B5 were considered appropriate for further investigation. 2B6 and 2B7 (micronised Bismuth Subnitrate) were excluded on the basis of there being more irritant that the other formulas tested and having no significant advantages. Thus, it is concluded that PE-LP gels are viable candidates for sealing teats. In vitro studies have shown that PE-LP gels have high tensile strengths for relatively low yield values. The merits of these two properties were studies by X-ray analysis of various PE-LP formulations in dry cows. A series of studies were undertaken to optimise these parameters:

## INJECTOR TYPE 2B - Study 1

| Formulation | Gel former % | LP % | Ac. % | BSN % | Mass g | P g/cm$^3$ | YV NM$^{-2}$ |
|---|---|---|---|---|---|---|---|
| 2B3 | 3.1% PE | 59.8 | 0.1 | 37 | 7.0 | 1.32 | 136.6 |
| 2B2 | 1.9% PE | 61.0 | 0.1 | 37 | 7.0 | 1.32 | 40.0 |
| 2B3[1] | 3.1% PE | 59.8 | 0.1 | 37 | 7.0 | 1.32 | 220.3 |

PE = Polyethylene
LP = Liquid Paraffin
AC = Acriflavin
BSN= Bismuth Subnitrate
P = Density
YV = Yield Value
2B3[1]= Formulation is identical to 2B3. Gel was formed using different temperature profile, leading to different yield values.

Each of the test formulations was infused into quarters of dry cows. The effectiveness of sealing was measured by X-ray analysis. In addition to mass of seal recovered, % BSN recovered and the effective seal duration [ESD] were estimated.

| Formulation | ESD (days) | Mass of Seal recovered (%) | BSN recovered |
|---|---|---|---|
| 2B3 | 47.3 ± 16.0 | 0.62 (8.81) | 40.8 |
| 2B2 | 54.1 ± 10.4 | 3.00 (43.0) | 40.0 |
| 2B3[1] | 49.0 ± 12.9 | 1.38 (19.7) | 44.6 |

There was a direct correlation between the ESD and the mass of seal recovered for these products.

## INJECTOR TYPE 2B- Study 2

The effect of the product volume and density was examined in another series of X-ray studies.

| Formulation | Gel former % | LP % | Ac. % | BSN % | Mass g | P g/cm$^3$ | YV NM$^{-2}$ |
|---|---|---|---|---|---|---|---|
| 2B8 | 1.7% PE | 33.2 | 0.1 | 65 | 5.0 | 1.70 | 216.3 |
| 2B9 | 1.7% PE | 33.2 | 0.1 | 65 | 10.0 | 1.70 | 216.3 |

The results of these studies are shown in table below:

| Formulation | Mass (g) * recovered (%) | % BSN ** recovered |
|---|---|---|
| 2B8 | 3.78 (75.6) | 85.6 |
| 2B9 | 3.92 (39.2) | 92.2 |

\* Adjusted for water content
\*\* % BSN were greater than in the previous study indicating a greater integrity of seal. As this trial was of a shorter duration than study 1, this may have been a contributing factor. However, increasing the density and reducing the volume had a clearly beneficial effect on the product performance.

### INJECTOR TYPE 2B - Study 3

As it is intended to use the teat seal in conjunction with an antibiotic preparation this study looked at the effect of an aqueous and oily based antibiotic suspension on seal integrity over time. Additionally, the mass of seal was reduced to 3.5g to see if this could further enhance the seals effectiveness.

Products used are given in the table below:

| Formulation | Gel former % | LP % | Ac. % | BSN % | Mass g | P g/cm$^3$ | YV NM$^{-2}$ | Antibiotic |
|---|---|---|---|---|---|---|---|---|
| 2B8 | 1.7% PE | 33.2 | 0.1 | 65 | 3.5 | 1.70 | 216.3 | Oily |
| 2B9 | 1.7% PE | 33.2 | 0.1 | 65 | 3.5 | 1.70 | 216.3 | Aq. |

The products were then examined for their effective seal duration [ESD].

| Formulation | ESD (days) | Recovery of material (g) | BSN Recovery % | BSN Recovery in fluid proportion ppm |
|---|---|---|---|---|
| B8 | 56.8 ± 13.9 | 0 | 0 | 1200 |
| B9 | 60.3 ± 8.2 | 0.793 | 77.6 | 147 |

A combination of seal and a non aqueous based antibiotic offers a superior combination to seal and an oily based antibiotic. This can be deduced from the fact that the ESD's for the seal/aqueous combinations were significantly better than those for oily combinations. This is further evidenced by the high BSN recoveries (>75%) for the aqueous products, indicating retention of seal in situ. In contrast the amount of bismuth found in the fluid portion at parturition was far lower for the aqueous than the oily combination. This shows that there was a larger degree of dispersal of the seal into the udder with the oily combinations.

In conclusion, the use of polyethylene as a gelling agent in combination with heavy metal salts in a teat seal product in combination with an aqueous based antibiotic system provides a product with the desired properties as earlier outlined which should be efficacious in the treatment and prophylaxis of dry cow mastitis.

**11**

PREFERRED METHOD OF MANUFACTURE FOR INJECTOR TYPE 2B

|  | Gm/Kg |
| --- | --- |
| H.D.P.E. | 17.00 |
| Heavy Liquid Paraffin | 332.00 |
| Bismuth Subnitrate | 650.00 |
| Acriflavin (Pigment) | 0.994 |

Each injector contains 3.5g.

(1) Place heavy liquid paraffin in reactor vessel. Heat to 160°C for one hour. Cool to 40°C.

(2) Start emulsifiers and mixers and add the H.D.P.E. Heat to 145°C $\pm$ 5°C and maintain for one hour. Cool to 40°C.

(3) Add and blend the Bismuth Subnitrate and Acriflavin.

(4) Fill 3.5g into intramammary injector.

In vitro work has shown that polyethylene based gels have tensile strengths and yield values that may make them suitable candidates as sealing agents with low levels or possibly zero levels of heavy metal salts. One such injector type 2C may be prepared as follows:

INJECTOR TYPE 2C

|  | g/Kg |
| --- | --- |
| Polyethylene | 30.000 |
| Liquid Paraffin | 969.000 |
| Acriflavin | 1.000 |

1) Mix Polyethylene Beads and Liquid Paraffin for 20 minutes.

2) Heat mixture 140°C $\pm$ 5°C for one hour with continuous stirring.

3) Allow to cool to room temperature whilst stirring.

4) Add and blend to Acriflavin.

5) Fill 3.5g into intramammary injectors.

**Claims**

1. A veterinary composition for intramammary use in non-human animals comprising cloxacillin benzathine as an antibacterial agent characterised in that the composition comprises 600 mg of cloxacillin (as cloxacillin benzathine) per unit dose having an average dimension of less than 25 microns in an aqueous suspension including a suspension aid, a buffer, and a surfactant.

2. A veterinary composition as claimed in claim 1 wherein a substantial proportion of the antibiotic has an average dimension of less than 10µ.

3. A veterinary composition as claimed in claim 1 or 2 wherein the suspension aid is polyvinylpyrollidone.

4. A composition as claimed in any of claims 1 to 3 wherein the buffer is sodium citrate.

5. A veterinary composition as claimed in any preceding claim in the form of an injector for intramammary administration.

6. A veterinary composition as claimed in any preceding claim for use in the prophylaxis or treatment of mammary disorders in lactating animals.

7. A veterinary product for intramammary use in non-human animals during an animals' dry period, the product comprising a veterinary composition as claimed in any of claims 1 to 5 and a gel base seal formulation.

8. A veterinary composition as claimed in claim 7 wherein a heavy metal salt is present in the gel base, preferably in an amount of at least 40% by weight of the base, most preferably in an amount of between 50% and 75% by weight of the base, especially in an amount of approximately 65% by weight of the base.

9. A veterinary composition as claimed in claim 8 wherein the salt is bismuth sub-nitrate.

10. A veterinary composition as claimed in any of claims 7 to 9 wherein the base is a gel based on aluminium stearate.

11. A veterinary composition as claimed in claim 10 wherein the gel includes a vehicle such as liquid paraffin.

12. A veterinary composition as claimed in any of claims 7 to 9 wherein the gel comprises a polyethylene gel, either a low density polyethylene or a high density polyethylene.

**Patentansprüche**

1. Veterinärmedizinische Zusammensetzung für die intramammäre Anwendung bei nichthumanen Tieren, die Cloxacillin-Benzathin als ein antibakterielles Mittel umfaßt, dadurch gekennzeichnet, daß die Zusammensetzung 600 mg Cloxacillin (als Cloxacillin-Benzathin) pro Einzeldosis mit einer durchschnittlichen Größe von weniger als 25 $\mu$m in einer wäßrigen Suspension, einschließlich eines Suspensionshilfsmittels, eines Puffers und eines Tensids umfaßt.

2. Veterinärmedizinische Zusammensetzung nach Anspruch 1, worin ein wesentlicher Anteil des Antibiotikums eine durchschnittliche Größe von weniger als 10 $\mu$m aufweist.

3. Veterinärmedizinische Zusammensetzung nach Anspruch 1 oder 2, worin das Suspensionshilfsmittel Polyvinylpyrollidon ist.

4. Zusammensetzung nach einem der Ansprüche 1 bis 3, worin der Puffer Natriumcitrat ist.

5. Veterinärmedizinische Zusammensetzung nach einem der vorangehenden Ansprüche in der Form einer Injektionsvorrichtung zur intramammären Verabreichung.

6. Veterinärmedizinische Zusammensetzung nach einem der vorangehenden Ansprüche zur Anwendung bei der Prophylaxe oder Behandlung mammärer Erkrankungen bei laktierenden Tieren.

7. Veterinärmedizinisches Produkt für die intramammäre Anwendung bei nichthumanen Tieren während der Trockenperiode eines Tieres, wobei das Produkt eine veterinärmedizinische Zusammensetzung nach einem der Ansprüche 1 bis 5 und eine Abdichtungsformulierung auf Gelgrundlage umfaßt.

8. Veterinärmedizinische Zusammensetzung nach Anspruch 7, worin ein Schwermetallsalz in der Gelgrundlage vorliegt, bevorzugt in einer Menge von mindestens 40 Gew.-% bezogen auf die Grundlage, am meisten bevorzugt in einer Menge zwischen 50 Gew.-% und 75 Gew.-% bezogen auf die Grundlage, besonders in einer Menge von circa 65 Gew.-% bezogen auf die Grundlage.

9. Veterinärmedizinische Zusammensetzung nach Anspruch 8, worin das Salz Wismutsubnitrat ist.

**10.** Veterinärmedizinische Zusammensetzung nach einem der Ansprüche 7 bis 9, worin die Grundlage ein auf Aluminiumstearat basierendes Gel ist.

**11.** Veterinärmedizinische Zusammensetzung nach Anspruch 10, worin das Gel ein Vehikel, wie zum Beispiel flüssiges Paraffin einschließt.

**12.** Veterinärmedizinische Zusammensetzung nach einem der Ansprüche 7 bis 9, worin das Gel ein Polyethylengel, entweder ein Polyethylen mit niederer Dichte (PE-ND) oder ein Polyethylen mit hoher Dichte (PE-HD) umfaßt.

**Revendications**

**1.** Une composition vétérinaire destinée à l'emploi intramammaire chez les animaux non humains comprenant de la benzathine cloxacilline comme agent anti-bactérien, caractérisée en ce que la composition comprend 600 mg de cloxacilline (sous forme de benzathine cloxacilline) par dose unitaire ayant une dimension moyenne de moins de 25 microns dans une suspension aqueuse incluant une aide de suspension, un tampon et un tensioactif.

**2.** Une composition vétérinaire telle que revendiquée dans la revendication 1, dans laquelle une proportion substantielle de l'antibiotique a une dimension moyenne de moins de 10 $\mu$.

**3.** Une composition vétérinaire telle que revendiquée dans la revendication 1 ou 2, dans laquelle l'aide de suspension est de la polyvinylpyrrolidone.

**4.** Une composition telle que revendiquée dans l'une quelconque des revendications 1 à 3, dans laquelle le tampon est du citrate de sodium.

**5.** Une composition vétérinaire telle que revendiquée dans l'une quelconque des revendications précédentes, sous la forme d'un injecteur pour administration intramammaire.

**6.** Une composition vétérinaire telle que revendiquée dans l'une quelconque des revendications précédentes, destinée à être utilisée comme prophylaxie ou comme traitement de toubles mammaires chez des animaux en lactation.

**7.** Un produit vétérinaire pour emploi intramammaire chez des animaux non humains pendant la période sèche des animaux, le produit comprenant une composition vétérinaire telle que revendiquée dans l'une quelconque des revendications 1 à 5 et une formule d'obturation à base de gel.

**8.** Une composition vétérinaire telle que revendiquée dans la revendication 7, dans laquelle un sel de métal lourd est présent dans la base de gel, de préférence selon une quantité d'au moins 40% par poids de la base, encore plus préférablement selon une quantité d'entre 50% et 75% par poids de la base, spécialement selon une quantité d'environ 65% par poids de la base.

**9.** Une composition vétérinaire telle que revendiquée dans la revendication 8, dans laquelle le sel est du sous-nitrate de bismuth.

**10.** Une composition vétérinaire telle que revendiquée dans l'une quelconque des revendications 7 à 9, dans laquelle la base est un gel basé sur du stéarate d'aluminium.

**11.** Une composition vétérinaire telle que revendiquée dans la revendication 10, dans laquelle le gel inclut un véhicule tel que de la paraffine liquide.

**12.** Une composition vétérinaire telle que revendiquée dans l'une quelconque des revendications 7 à 9, dans laquelle le gel comprend un gel de polyéthylène, soit du polyéthylène basse densité, soit du polyéthylène haute densité.

Bioavailability of Cloxacillin in Milk: Data for
Individual Quarters(n=30) in Eight Cows following
infusion of injector 1A(Aqueous Formulation)

$y = 1011 \cdot 0^{\cdot} 10^{(-5 \cdot 5859e-2X)}$
$R^2 = 0.912$

Fig. 1.1

Bioavailability of Cloxacillin in Milk: Data for individual Quarters (n=30) in Eight Cows following infusion of injector 1A (Aqueous Formulation)

$$y = 1011.0 * 10^{(-5.5859e-2X)}$$
$$R^2 = 0.912$$

Cloxacillin (mgil) in Milk

Time Post Infusion (Hours)

917824-72h Graph

Fig. 1.2

Bioavailability of Cloxacillin in Milk : Data for
individual Quarters (n= 30) in Eight Cows following
infusion of injector 1A (Aqueous Formulation) –
Terminal Elimination Phase

9178 Term. Elm. Graph

Fig. 1.3

$$y = -121x + 6.848, r^2 = .994$$

O ln(x) of Cloxacillin (mgil)

Fig. 2